# EUROPEAN PATENT APPLICATION

(11) **EP 3 147 516 A1**
(43) Date of publication of application: **29.03.2017**
(21) Application number: 16192846.0
(22) Date of filing: 03.09.2013
(51) Int. Cl.: F04D 29/44, A61M 16/00, F04D 29/28, F04D 29/42, F04D 25/08, F04D 29/66

(54) **BLOWER**

(30) Priority: 03.09.2012 JP 2012193041
(62) Divisional of application: 13832856.2
(71) Applicant: Metran Co., Ltd., Kawaguchi-shi, Saitama 332-0015 (JP)
(72) Inventor: NITTA, Kazufuku, Saitama, 332-0015 (JP); SHIOTA, Shinichi, Saitama, 332-0015 (JP); HIGASHIURA, Masashi, Tokyo, 156-0043 (JP)
(74) Representative: TBK

(57) **Abstract**

The invention pertains to a blower (10). The blower (10) comprises a housing (11) having an intake port (16) and a flow passage (22) that communicates with a discharge port (17); an impeller (13) disposed in the housing (11) so as to have a front surface facing the intake port (16); and a partitioning member (15) that is disposed toward a rear surface of the impeller (13) to partition an inside of the housing (11) into a space (21) in which the impeller (13) is disposed and the flow passage (22). The partitioning member (15) forms a slit (d), which extends along an inner peripheral surface of the housing (11), between the partitioning member (15) and the inner peripheral surface of the housing (11). The slit (d) has a width (w) of 0.6 mm or less.

## Description

### Technical Field

The present invention relates to a blower for use in respiration assistance devices.

### Background Art

Sleep apnea is caused by the airway muscle being relaxed during sleep, so that the tongue base and the soft palate are lowered to thereby block the airway. For those patients of this type of respiration troubles, such a respiration assistance device is employed which applies a positive pressure to the airway (see Japanese Patent Application Laid-Open No. 2012-115375 and a non-patent document (Metran Co., Ltd., [online], product information > Jusmine, [accessed on June 29, 2012], the Internet (URL: http://www.metran.co.jp/products/products2/190.html))). The respiration assistance device requires a pump unit for producing a positive pressure in the airway. Employed as a power source for the pump unit is, for example, a blower for rotating impellers (fans) to transfer a gas.

### Summary of Invention

### Technical Problem

Such a respiration assistance device, which supports comfortable sleep, is preferably as quiet as possible, more preferably, ultimately silent. It is thus desired to provide a silenced blower which would otherwise be a noise source.

The present invention has been developed in view of the aforementioned problem. It is thus an object of the present invention to provide a blower which implements silencing. Solution to Problem

The present invention provides a blower including: a housing having an intake port and a flow passage that communicates with a discharge port; an impeller disposed in the housing so as to have a front surface facing the intake port; and a partitioning member that is disposed toward the rear surface of the impeller to partition the inside of the housing into a space in which the impeller is disposed and the flow passage. The partitioning member forms a slit, which has a width of 1.0 mm or less and extends along the inner peripheral surface of the housing, between the partitioning member and the inner peripheral surface of the housing.

According to the present invention, since a two-stage structure with the space in which the impeller is disposed and the flow passage that communicates with the discharge port is employed, it is possible to separate the flow of air drawn through the intake port from the flow of air delivered through the discharge port. This makes it possible to prevent the flow of air drawn through the intake port from colliding with the flow of air delivered through the discharge port. That is, it is possible to prevent the occurrence of noise due to the flows of air colliding with each other.

Furthermore, air flowing through the slit connecting between the space in which the impeller is disposed and the flow passage causes noise to occur. This noise can cancel out another noise that is caused by the rotation of the impeller.

Furthermore, according to the present invention, the blower of the aforementioned means is characterized in that the flow passage extends along the slit.

Furthermore, according to the present invention, the blower of the aforementioned means is characterized in that the slit has a width of 1.5% or less of the inner peripheral diameter of the housing.

Furthermore, according to the present invention, the blower of the aforementioned means is characterized in that the flow passage has a sliced sectional shape which is a perfect circle or a relatively elongated shape in the radial direction of the impeller.

According to the aforementioned invention, the air flowing into the flow passage from the space in which the impeller is disposed is smoothly moved along the sidewall, the bottom surface, and the ceiling surface of the flow passage. This prevents the occurrence of a turbulent flow. In turn, the occurrence of noise is prevented.

Furthermore, according to the present invention, the blower of the aforementioned means is characterized by including a motor for rotating the impeller.

Furthermore, according to the present invention, the blower of the aforementioned means is characterized in that the impeller, the motor, and the flow passage are disposed in that order in a direction along the rotation axis of the impeller.

Furthermore, according to the present invention, the blower of the aforementioned means is characterized in that the flow passage is disposed so as to orbit around the motor.

According to the aforementioned invention, it is possible to reduce the size of the blower in the direction of the rotational axis of the impeller.

Furthermore, according to the present invention, the blower of the aforementioned means is characterized by including a rectifying member that is provided at the center of the intake port so as to be protruded outwardly from the intake port.

According to the aforementioned invention, when compared with the case with no rectifying member included and with the rectifying member not protruded outwardly from the intake port but accommodated therein, it is possible to prevent the collision of air near the intake port. For example, in the case with no rectifying member included, the air drawn through the intake port collides with the impeller and the rotation axis thereof. However, the aforementioned invention will never cause such a collision. On the other hand, with the rectifying member not protruded outwardly from the intake port but accommodated therein, the rectifying member abruptly narrows the inside of the housing and thereby causes the collision of the air drawn into the housing. However, the aforementioned invention will never cause such a collision. This makes it possible to prevent the occurrence of noise.

Furthermore, according to the present invention, the blower of the aforementioned means is characterized in that the rectifying member supports one end of the rotation axis of the impeller.

According to the aforementioned invention, the vibration of the impeller can be prevented. In turn, it is possible to prevent the occurrence of noise. Furthermore, since the rectifying member also serves to support one end of the rotation axis of the impeller, the count of parts can be reduced, thereby reducing the weight and the size of the blower. This allows one to carry the blower readily for travel or a business trip with an overnight stay.

Furthermore, according to the present invention, the blower of the aforementioned means is characterized in that the impeller includes a plurality of blades disposed around the rotation axis and a cover member for covering the plurality of blades toward the partitioning member, with the plurality of blades being open toward the intake port.

According to the aforementioned invention, when compared with the case where the cover member is included to cover the plurality of blades toward the intake port, it is possible to reduce the weight and the size of the blower. This allows one to carry the blower readily for travel or a business trip with an overnight stay.

Furthermore, according to the present invention, the blower of the aforementioned means is characterized in that the cover member has a cone surface protruded toward the intake port.

According to the aforementioned invention, the air drawn into the housing through the intake port can smoothly flow along the cover member. This makes it possible to prevent the air drawn into the housing through the intake port from colliding with the cover member. In turn, it is possible to prevent the occurrence of noise.

Furthermore, according to the present invention, the blower of the aforementioned means is characterized by being used for a respiration assistance device.

According to the aforementioned invention, since silencing can be implemented, it is possible to reduce an auditory burden on the patient who uses the respiration assistance device. That is, it is possible to prevent the patient who uses the respiration assistance device from being disturbed during sleep.

Furthermore, according to the present invention, the blower of the aforementioned means includes: a housing having an intake port and a flow passage that communicates with a discharge port; an impeller disposed in the housing so as to have a front surface facing the intake port; and a partitioning member that is disposed toward the rear surface of the impeller to partition the inside of the housing into the space in which the impeller is disposed and the flow passage. The partitioning member forms a slit, which extends along the inner peripheral surface of the housing, between the partitioning member and the inner peripheral surface of the housing. The partitioning member is provided with an air passage connecting between the space and the flow passage.

According to the aforementioned invention, since a two-stage structure with the space in which the impeller is disposed and the flow passage that communicates with the discharge port is employed, it is possible to separate the flow of air drawn through the intake port from the flow of air delivered through the discharge port. This makes it possible to prevent the flow of air drawn through the intake port from colliding with the flow of air delivered through the discharge port. That is, it is possible to prevent the occurrence of noise due to the flows of air colliding with each other.

Furthermore, air flowing through the slit connecting between the space in which the impeller is disposed and the flow passage causes noise to occur. This noise can cancel out another noise that is caused by the rotation of the impeller.

Furthermore, since the partitioning member is provided with the air passage, the patient's breath flowing back to the flow passage flows through the air passage and escapes through the flow passage. This prevents the patient's breath flowing back to the flow passage from colliding (so-called "fighting") near the slit with the air flowing into the flow passage via the slit (the air to be breathed by the patient.) That is, it is possible to prevent the occurrence of noise caused by the breath and the intake air colliding with each other. Furthermore, since the fighting is prevented, it is prevented to exhale a breath with difficulty.

Furthermore, according to the present invention, the blower of the aforementioned means is characterized in that the flow passage extends along the slit.

Furthermore, according to the present invention, the blower of the aforementioned means is characterized in that the air passage is formed so as to face the rear surface of the impeller.

The centrifugal force arising from the rotation of the impeller creates a negative pressure in the gap between the rear surface of the impeller and the partitioning member. According to the aforementioned invention, it is possible to pull the patient's breath flowing back to the flow passage into the air passage by the negative pressure created by the centrifugal force due to the rotation of the impeller. This ensures that the patient's breath flowing back to the flow passage is prevented from colliding near the slit with the air flowing into the flow passage via the slit (the air to be breathed by the patient).

Furthermore, according to the present invention, the blower of the aforementioned means is characterized in that the slit has a width of 1.0 mm or less.

### Advantageous Effects of Invention

The present invention can provide outstanding effects of implementing silencing.

### Brief Description of Drawings

FIG. 1 is a top view illustrating a blower according to a first embodiment of the present invention.
FIG. 2 is a front view illustrating the blower shown in FIG. 1.
FIG. 3 is a longitudinal sectional view illustrating the blower shown in FIG. 1.
FIG. 4 is a top view illustrating an impeller.
FIG. 5 is a cross-sectional view illustrating a flow passage.
FIG. 6 is a graph showing the relation between the width of a slit extending along the inner peripheral surface of the housing and the noise cancel level, with the horizontal axis representative of the width of the slit and the vertical axis representative of the noise cancel level.
FIG. 7 is a longitudinal sectional view illustrating a blower according to a comparative example.
FIG. 8 is a view illustrating a blower according to a second embodiment of the present invention, (A) a longitudinal sectional view and (B) a top view illustrating an impeller and a partitioning member.
FIG. 9 is a view illustrating a blower according to a third embodiment of the present invention, (A) a longitudinal sectional view and (B) a top view of an impeller and a partitioning member.

### Description of Embodiments

Now, referring to the drawings, a blower according to the present invention will be described in more detail.

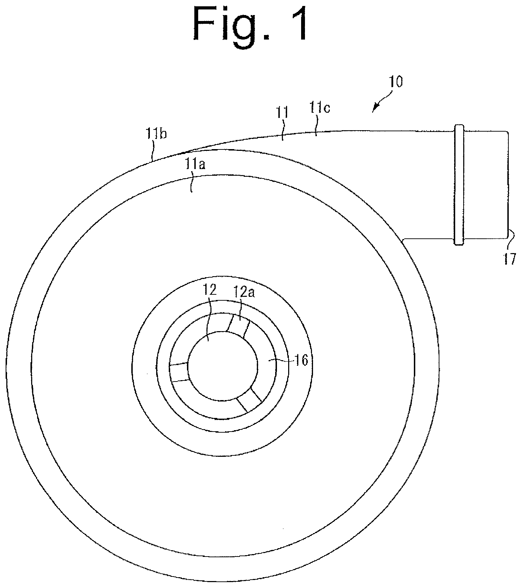

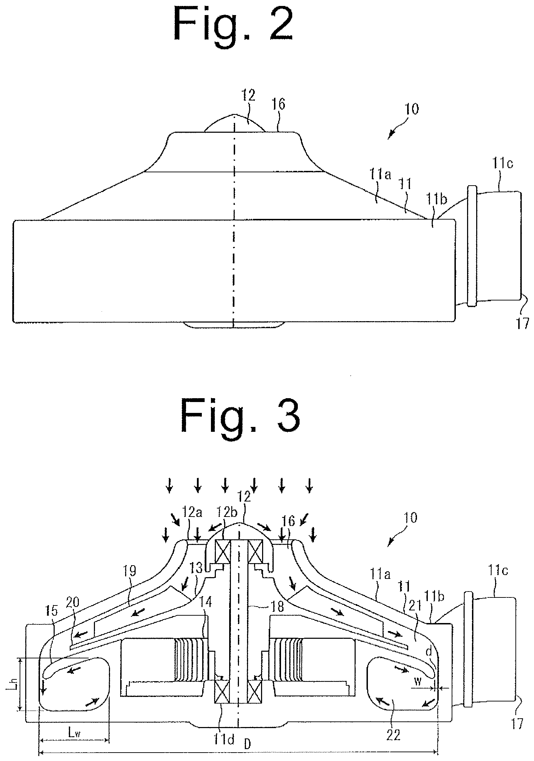

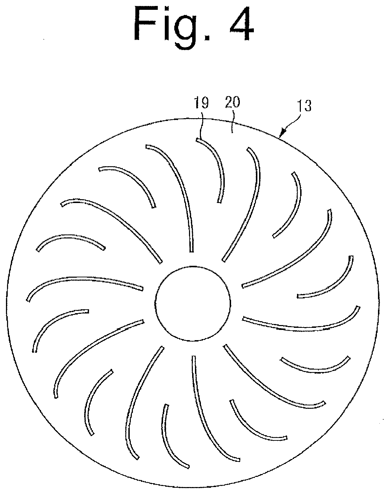

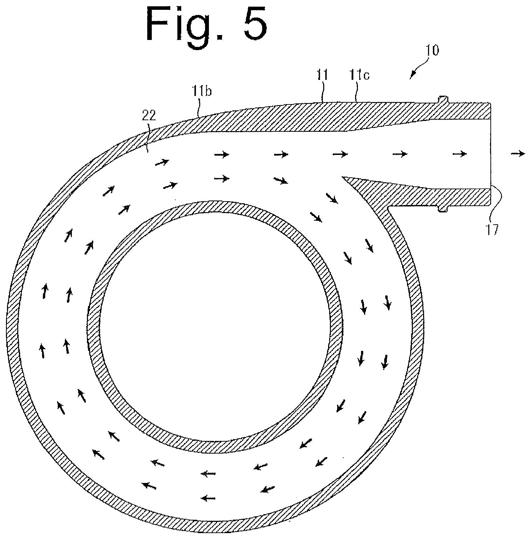

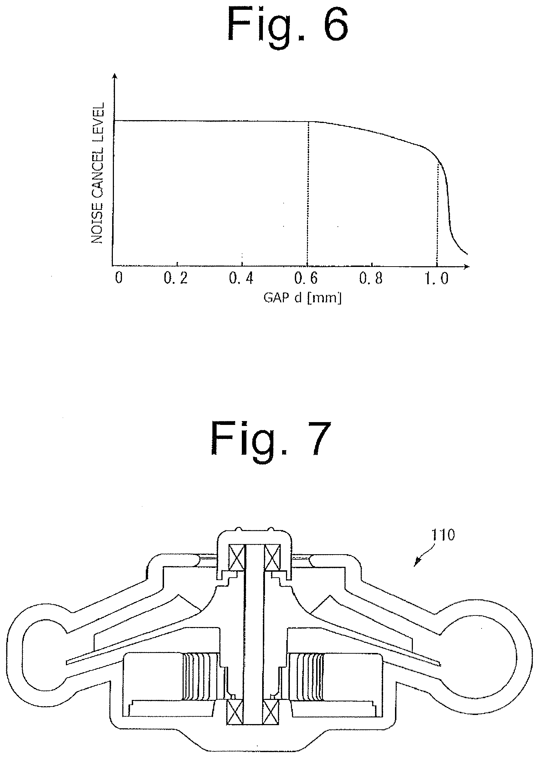

First, referring to Figs. 1 to 6, a description will be made to the configuration of a blower 10 according to a first embodiment of the present invention. FIG. 1 is a top view of the blower 10. FIG. 2 is a front view of the blower 10. FIG. 3 is a longitudinal sectional view of the blower 10. FIG. 4 is a top view of an impeller 13. FIG. 5 is a cross-sectional view of a flow passage 22. FIG. 6 is a graph showing the relation between the width w of a slit d extending along (orbiting around) the inner peripheral surface of a housing 11 and the noise cancel level, with the horizontal axis representative of the width w of the slit d and the vertical axis representative of the noise cancel level. Note that throughout the drawings, for example, part of the structures and hatching for showing cross-sections may be omitted, as appropriate, to simplify the drawings. Furthermore, in each figure, the size of the members may be exaggerated as appropriate.

The blower 10 shown in FIG. 1 to FIG. 3, which is a power source for a pump unit that is required for a respiration assistance device used by a patient having a respiratory Problem, blows air into the airway to create a positive pressure. Note that the pump unit is provided, as appropriate, with one or more blowers 10. Furthermore, the respiration assistance device is connected, as appropriate, with one or more pump units.

Specifically, the blower 10 includes the housing 11, a rectifying member 12, the impeller 13, a motor 14, and a partitioning member 15.

The housing 11, which is the main body of the blower 10 molded of a resin, is made up of an upper part 11a having a generally truncated cone outer shape, a lower part 11b having a generally cylindrical outer shape, and a discharge pipe 11c extended sideward from the lower part 11b. The upper part 11a is smoothly curved upwardly. Furthermore, the upper part 11a has a circular intake port 16 at the upper end. In the lower part 11b a bearing 11d which functions as a bearing for supporting the rotation axis 18 of the impeller 13 is embedded. The discharge pipe 11c has, at the tip, a discharge port 17. Such a housing 11 is configured to take in air through the intake port 16 and take out the air through the discharge port 17. Note that the invention is not limited to air, but may also be applicable to air mixed with a chemical or another gas such as oxygen.

The rectifying member 12 is designed to resemble the gas turbine type jet engine so as to have a shape with the tip end protruded. The rectifying member 12 is provided at the center of the intake port 16 so as to be protruded outwardly from the intake port 16. Then, the rectifying member 12 is securely coupled to the rim of the intake port 16, for example, by three coupling members 12a. Furthermore, the rectifying member 12, which has a bearing 12b embedded therein, also serves as a bearing for supporting the rotation axis 18 of the impeller 13.

The impeller 13 shown in FIG. 3 and FIG. 4 is disposed within the housing 11 so that the front surface thereof faces the intake port 16. That is, the impeller 13 is located closer to the intake port 16 than to the flow passage 22, to be discussed later, in the direction of the rotation axis 18. The impeller 13 includes a plurality of blades 19 disposed around the rotation axis 18 and a cover member 20 that covers the rear surface of the plurality of blades 19 (the lower side in FIG. 3). Then, the impeller 13 is configured such that the plurality of blades 19 toward the intake port 16 are open. That is, the plurality of blades 19 toward the intake port 16 are not provided with any member such as the cover member 20. The plurality of blades 19 are molded integrally with the cover member 20.

The plurality of blades 19 face the inner peripheral surface of the housing 11. Furthermore, if it is possible to make the plurality of blades 19 as designed, then the gap between the inner peripheral surface of the housing 11 and the blades 19 is preferably as close to 0 mm as possible. However, in consideration of a design error, from the viewpoint of preventing a collision between the blades 19 and the inner peripheral surface of the housing 11, it is preferable to provide a certain gap (a gap having the same magnitude as that of the design error: a gap of 0.8 mm for the design error being ± 0.8 mm.) The cover member 20 exhibits the shape of an umbrella that protrudes toward the intake port 16. That is, the cover member 20 has a cone surface that is protruded toward the intake port 16. This causes a space for accommodating the motor 14 or the like to be formed toward the rear surface (the lower side in FIG. 3) of the cover member 20. The rotation axis 18 of the impeller 13 is supported at respective ends by the bearing 11d embedded in the housing 11 and a bearing 12b embedded in the rectifying member 12.

The motor 14 shown in FIG. 3 is provided so as to be slightly accommodated toward the rear surface (the lower side in FIG. 3) of the impeller 13 (the cover member 20). The motor 14 is a power source for rotating the impeller 13 about the rotation axis 18. Typically, the number of revolutions is preferably about 10000 [rpm] to 20000 [rpm].

The partitioning member 15 is disposed toward the rear surface of the impeller 13, thereby partitioning the inside of the housing 11 into a space 21 in which the impeller 13 is disposed and the flow passage 22 that communicates with the discharge port 17. The partitioning member 15 forms the slit d with a width w equal to 1.0 mm or less that extends along the inner peripheral surface of the housing 11 between the partitioning member 15 and the inner peripheral surface of the housing 11. The slit d is preferably 0.6 mm or less from the viewpoint of reducing noise, and more preferably the maximum value or 0.6 mm from the viewpoint of reducing the loss of energy. As shown in FIG. 6, this is because the slit d greater than 0.6 mm would cause the noise cancel level to be gradually reduced, and the slit d greater than 1.0 mm would cause the noise cancel level to be abruptly reduced. Then, this is because the narrower the slit d is, the greater the loss of energy becomes. Note that the slit d preferably have a width w that is 1.5% or less of the inner peripheral diameter D of the housing 11.

The flow passage 22 shown in FIG. 3 and FIG. 5 is disposed in a concentrically annular shape (in an annular shape having a constant cross-sectional area) so as to extend along the slit d and orbit around the motor 14, and then communicates with the discharge pipe 11c. Then, the flow passage 22 is provided slightly below the motor 14 (below in FIG. 3) so that the impeller. 13, the motor 14, and the flow passage 22 are disposed in that order in the direction along the rotation axis 18 of the impeller 13. The flow passage 22 has most preferably a perfect circular sliced sectional shape, and preferably a relatively elongated shape in the radial direction of the impeller 13 (the horizontal direction in FIG. 3.) In this embodiment, to reduce the size (the thickness) in the direction of height (in the vertical direction in FIG. 3), the flow passage 22 has, as a sliced sectional shape, a relatively elongated shape in the radial direction of the impeller 13 (L_{w} > Lₕ in FIG. 3.) Furthermore, the flow passage 22 is preferably set so that the sliced cross-sectional area is as large as possible.

Now, referring to Figs. 3 and 5, a description will be made to the flow of air through the blower 10. Note that the air flow that serves to be breathed by the patient is denoted by black bold arrows.

As shown in FIG. 3, rotating the impeller 13 will cause the air inside the space 21, in which the impeller 13 is disposed, to be moved in the outer circumferential direction (in the sideward direction of FIG. 3.) This in turn causes a drop in atmospheric pressure toward the inner periphery

(toward the center in FIG. 3) of the space 21 in which the impeller 13 is disposed. This results in the air being drawn into the space 21 inside the housing 11 through the intake port 16. In other words, there occurs an air current through the intake port 16 towards the slit d inside the housing 11.

Furthermore, the air inside the space 21 in which the impeller 13 is disposed is moved in the outer circumferential direction (in the sideward direction of FIG. 3), thereby causing a rise in atmospheric pressure toward the outer periphery of the space 21 (toward the right and left in FIG. 3.) This results in the air inside the space 21, in which the impeller 13 is disposed, being moved from the slit d Loward the flow passage 22. That is, there occurs an air current from the space 21, in which the impeller 13 is disposed, toward the flow passage 22.

Then, the air moved from the slit d toward the flow passage 22 is moved along the wall surface, the bottom surface, and the ceiling surface of the flow passage 22. Furthermore, the air moved from the slit d toward the flow passage 22 is subjected to the force that causes the air to be rotated in the rotational direction of the impeller 13 (in a clockwise direction) due to the rotation of the impeller 13. Thus, as shown in FIG. 5, the air moved from the slit d toward the flow passage 22 is rotated in a clockwise direction inside the flow passage 22. Subsequently, the air rotated in a clockwise direction inside the flow passage 22 is delivered through the discharge port 17 through the discharge pipe 11c.

Now, a description will be made to Experiment 1 to Experiment 4 in that order.

Experiment 1 and Experiment 2 were carried out for a comparison with a blower 110 shown in FIG. 7 that serves as a master. Like the blower 10 according to the embodiment above, the blower 110 serves as a power source for a pump unit that is required for a respiration assistance device used by a patient having a respiratory problem, and blows air into the airway to create a positive pressure.

Unlike the blower 10 according to the aforementioned embodiment, the blower 110 includes no rectifying member. On the other hand, the blower 110 is designed such that the gap between the blades and the inner peripheral surface of the housing is set to 1.9 [mm]. Furthermore, the blower 110 employs a single-stage structure configured such that the space in which the impeller is disposed is continuous to the flow passage that communicates with the discharge port. That is, the blower 110 does not includes a structure that is equivalent to the slit d of the blower 10 according to the aforementioned embodiment. Still furthermore, the blower 110 exhibits a scroll shape (spiral shape) configured such that the sliced cross-sectional area of the flow passage is gradually widened toward the discharge port. The blower 110 is also configured such that the impeller, the flow passage, and the motor are disposed in that order in the direction along the rotation axis of the impeller. Note that the blower 110 is employed for a continuous automatic airway positive pressure unit (trade name: Jusmine) sold by Metran Co., Ltd. (Kawaguchi City, Saitama prefecture.)

Experiment 3 was carried out to compare the blower 10 according to the aforementioned embodiment and a blower according to a variation thereof. Experiment 4 was carried out to compare the blower 10 according to the aforementioned embodiment with a product made by another manufacturer.
[Experiment 1] First, a description will be made to Experiment 1 in which noise reduction by the rectifying member was examined. Experiment 1 was conducted to compare the noise level [dB] between the blower 110 serving as the master and the blower 110 fitted with a rectifying member. As a result, fitting with the rectifying member leads to a reduction in the noise level by about 1.7 [dB] to 2.5 [dB]. At mid- and high-range frequencies equal to or greater than about 750 [Hz], the acoustic power [dB] was reduced.
[Experiment 2] Now, a description will be made to Experiment 2 which was conducted to examine the effects on noise by the gap between the blades and the inner peripheral surface of the housing. In Experiment 2, the noise level [dB] was compared between the blower 110 serving as the master and the blower 110 that is fitted with the rectifying member with the gap between the blades and the inner peripheral surface of the housing reduced to 0.8 [mm]. As a result, fitting with the rectifying member with the gap between the blades and the inner peripheral surface of the housing reduced to 0.8 [mm] lead to a reduction in noise level by about 3.8 [dB] to 4.2 [dB]. In particular, fitting with the rectifying member with the gap between the blades and the inner peripheral surface of the housing reduced to 0.8 [mm] leads to a reduction in the acoustic power [dB] at mid- and low-range frequencies equal to or less than about 3200 [Hz]. In consideration of Experiment 1, it can be seen that reducing the gap between the blades and the inner peripheral surface of the housing to 0.8 [mm] leads to a reduction in the acoustic power [dB] at mid- and low-range frequencies.
[Experiment 3] A description will also be made to Experiment 3 which was conducted to examine the effects on noise by the slit extending along the inner peripheral surface of the housing. Experiment 3 was conducted to compare the noise level [dB] between the blower 10 according to the aforementioned embodiment and the blower 10 with the width w of the slit d between the partitioning member 15 and the inner peripheral surface of the housing 11 changed to 1.5 [mm] or 2.0 [mm]. That is, the noise level [dB] was compared between each of the cases with the slit d being 1.0 [mm], 1.5 [mm], and 2.0 [mm]. As a result, reducing the width w of the slit d between the partitioning member 15 and the inner peripheral surface of the housing 11 to 1.0 [mm] leads to a reduction in the noise level by about 0.9 [dB] to 3.2 [dB] when compared with the cases of 1.5 [mm] and 2.0 [mm]. On the other hand, the slit d with the width w being 1.5 [mm] and 2.0 [mm] showed no significant change in the noise level therebetween. Reducing the width w of the slit d between the partitioning member 15 and the inner peripheral surface of the housing 11 to 1.0 [mm] leads to a reduction in the acoustic power [dB] generally throughout the entire region.
[Experiment 4] A description will also be made to Experiment 4 for a comparison of the noise level with a product supplied by another manufacturer. Experiment 4 was conducted to compare the noise level [dB] between the blower 10 according to the aforementioned embodiment and a blower (see Japanese Patent No. 4497809) employed for a continuous automatic airway positive pressure unit (trade name: S9Elite) sold by RESMED Limited (Australia), which has achieved the world's lowest noise level at the time of filing the subject application, and RESUMED in Japan (Bunkyo-ku, Tokyo). As a result, the blower 10 according to this embodiment had a noise level reduced by about 1.4 [dB] to 3.0 [dB] when compared with the aforementioned product by the manufacturer which has achieved the world's lowest noise level. The blower 10 according to this embodiment had a reduced acoustic power [dB] in a mid-range of about 750 [Hz] or higher and about 6400 [Hz] or lower when compared with the aforementioned product by the manufacturer which has achieved the world's lowest noise level.

As described above, the blower 10 employs the two-stage structure which is made up of the space 21 in which the impeller 13 is disposed and the flow passage 22 that communicates with the discharge port 17, and thus can separate the flow of air drawn through the intake port 16 from the flow of air delivered through the discharge port 17. This makes it possible to prevent the flow of air drawn through the intake port 16 from colliding with the flow of air delivered through the discharge port 17. That is, it is possible to prevent the occurrence of noise that may be caused by the flows of air colliding with each other.

On the other hand, the air flowing through the slit d communicating between the space 21 in which the impeller 13 is disposed and the flow passage 22 may cause noise to occur. The noise can be used to cancel out another noise that is caused by the rotation of the impeller 13.

Then, the air having flown from the space 21 in which the impeller 13 is disposed into the flow passage 22 is smoothly moved along the side surface, the bottom surface, and the ceiling surface of the flow passage 22. This prevents the occurrence of a turbulent flow. In turn, the occurrence of noise is prevented.

Furthermore, since the flow passage 22 is disposed so as to orbit around the motor 14, it is possible to reduce the size in the direction of the rotation axis 18 of the impeller 13.

Furthermore, since the rectifying member 12 is provided so as to be protruded outwardly from the intake port 16, the collision of air near the intake port 16 can be prevented when compared with the case where the rectifying member is not included and the case where the rectifying member is not protruded outwardly from the intake port 16 but accommodated therein. For example, with no rectifying member included, the air drawn through the intake port 16 collides with the impeller 13 and the rotation axis 18 thereof. However, the aforementioned blower 10 will never cause such a collision. On the other hand, with the rectifying member not protruded outwardly from the intake port 16 but accommodated therein, the rectifying member abruptly narrows the inside of the housing 11 and thereby causes a collision of the air drawn into the housing 11. However, the aforementioned blower 10 will never cause such a collision. This makes it possible to prevent the occurrence of noise.

Then, since the rectifying member 12 supports one end of the rotation axis 18 of the impeller 13, the vibration of the impeller 13 can be prevented. In turn, it is possible to prevent the occurrence of noise. Furthermore, since the rectifying member 12 also serves to support one end of the rotation axis 18 of the impeller 13, the count of parts can be reduced, thus reducing the weight and the size of the blower. This allows one to carry the blower readily for travel or a business trip with an overnight stay.

furthermore, since the impeller 13 is configured such that the plurality of blades 19 toward the intake port 16 arc open, it is possible to reduce the weight and the size when compared with the case where the cover member is included to cover the plurality of blades 19 toward the intake port 16. This allows one to carry the blower readily for travel or a business trip with an overnight stay.

Furthermore, since the cover member 20 has a cone surface protruding toward the intake port 16, it is possible to flow the air drawn through the intake port 16 into the housing 11 smoothly along the cover member 20. This makes it possible to prevent the air drawn through the intake port 16 into the housing 11 from colliding with the cover member 20. In turn, it is possible to prevent the occurrence of noise.

Then, since the aforementioned blower 10 can be silenced, it is possible to reduce an auditory burden on the patient who uses the respiration assistance device. That is, it is possible to prevent the patient who uses the respiration assistance device from being disturbed during sleep.

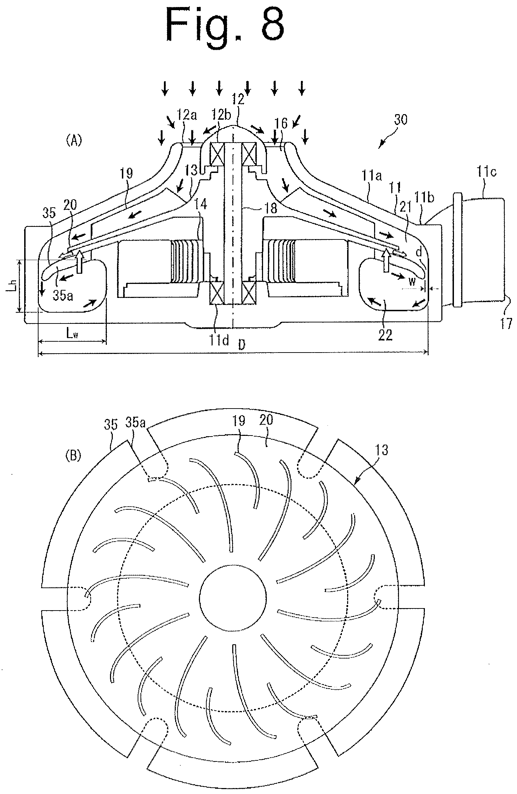

Now, referring to FIG. 8, a description will be made to the configuration of a blower 30 according to a second embodiment of the present invention. FIG. 8 (A) is a longitudinal sectional view illustrating the blower 30. FIG. 8(B) is a top view illustrating the impeller 13 and a partitioning member 35. Note that the flow of a patient's breath is denoted by hollow arrows.

Note that here, a description will be made only to the characteristic parts of the blower 30, and the description of the same configuration, operation, and effects as those of the blower 10 will be omitted as appropriate. Furthermore, for the third embodiment to be discussed below, a description will be made only to the characteristic parts.

As shown in Figs. 8(A) and 8(B), the blower 30 includes the partitioning member 35 in place of the partitioning member 15 of the first embodiment (see FIG. 3). The partitioning member 35 is provided with a plurality of air passages 35a so that the outer circumference is notched. More specifically, the partitioning member 35 is provided with six air passages 75a at equal intervals so that the outer circumference is notched. The plurality of air passages 35a couple between the space 21 and the flow passage 22. The plurality of air passages 35a serve to let the patient's breath flowing back to the flow passage 22 escape into the space 21 toward the impeller 13.

As described above, since the blower 30 is configured such that the air passages 35a are formed in the partitioning member 35, the patient's breath flowing back to the flow passage 22 is allowed to flow through the air passages 35a and then escape through the flow passage 22. This prevents the patient's breath flowing back through the flow passage 22 from colliding (so-called "fighting") near the slit d with the air flowing via the slit d into the flow passage 22 (the air to be breathed by the patient). That is, it is possible to prevent the occurrence of noise that is caused by the breath and the intake air colliding with each other. Furthermore, since the fighting is prevented, it is prevented to exhale a breath with difficulty.

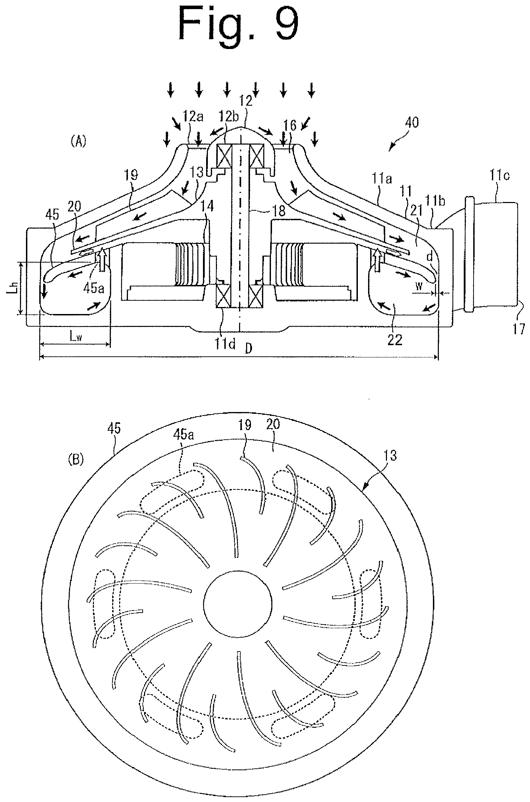

Now, referring to FIG. 9, a description will be made to the configuration of a blower 40 according to the third embodiment of the present invention. FIG. 9(A) is a longitudinal sectional view illustrating the blower 40. FIG. 9(B) is a top view of the impeller 13 and a partitioning member 45. Note that the flow of a patient's breath is denoted by hollow arrows.

As shown in Figs. 9(A) and 9(B), the blower 40 includes the partitioning member 45 in place of the partitioning member 15 (see FIG. 3) of the first embodiment. The partitioning member 45 is provided with a plurality of air passages 45a which are formed inwardly relative to the slit d and independently of the slit d so as to penetrate therethrough at positions along the inner circumference. More specifically, the partitioning member 45 is provided with six air passages 45a formed at equal intervals so as to penetrate therethrough at positions along the inner circumference. The plurality of air passages 45a couple between the space 21 and the flow passage 22. The plurality of air passages 45a are formed to face the rear surface of the impeller 13. Then, the plurality of air passages 45a serve to let the patient's breath flowing back to the flow passage 22 escape into the space 21 toward the impeller 13.

There occurs a negative pressure in the gap between the rear surface of the impeller 13 and the partitioning member 45 due to the centrifugal force produced by the rotation of the impeller 13. Thus, according to the blower 40, the negative pressure created by the centrifugal force due to the rotation of the impeller 13 can pull the patient's breath flowing back to the flow passage 22 into the air passages 45a. This ensures that the patient's breath flowing back to the flow passage 22 is prevented from colliding near the slit d with the air flowing into the flow passage 22 via the slit d (the air to be breathed by the patient).

The present invention is not limited to each of the aforementioned embodiments, but may also be modified in a variety of ways without departing from the gist and the technical idea of the invention.

That is, in each of the aforementioned embodiments, the position, magnitude (size), shape, material, orientation, and number of quantities of each component can be changed as appropriate. For example, the position, magnitude, shape, and number of quantities of the air passages 35a and 45a can be changed as appropriate. More specifically, the magnitude and the number of quantities of the air passages 35a and 45a may be set depending on the vital capacity of the patient.

That is, in the second and the third embodiments above, not only the partitioning members 35 and 45 are provided with the air passages 35a and 45a, but also the impeller 13 may be provided with air passages.

### Reference Signs List

- 10, 30, 40: blower
- 11: housing
- 12: rectifying member
- 13: impeller
- 14: motor
- 15, 35, 45: partitioning member
- 16: intake port
- 17: discharge port
- 18: rotation axis
- 19: blade
- 20: cover member
- 21: space
- 22: flow passage
- 35a, 45a: air passage
- w: width
- d: slit
- D: inner peripheral diameter

This application is a divisional application of European patent application no. 13 832 856.2 (the "parent application"), also published under no. EP-A- 2 889 490. The following items corresponding to the originally filed items of the parent application form part of the content of this description as filed.
1. A blower comprising:
   a housing having an intake port and a flow passage that communicates with a discharge port;
   an impeller disposed in the housing so as to have a front surface facing the intake port; and
   a partitioning member that is disposed toward a rear surface of the impeller to partition an inside of the housing into a space in which the impeller is disposed and the flow passage, wherein
   the partitioning member forms a slit, which has a width of 1.0 mm or less and extends along an inner peripheral surface of the housing, between the partitioning member and the inner peripheral surface of the housing.
2. The blower according to item 1, wherein
   the flow passage extends along the slit.
3. The blower according to item 1 or 2, wherein
   the slit has a width of 1.5% or less of an inner peripheral diameter of the housing.
4. The blower according to any one of items 1 to 3, wherein
   the flow passage has a sliced sectional shape which is a perfect circle or a relatively elongated shape in a radial direction of the impeller.
5. The blower according to any one of items 1 to 4, comprising a motor for rotating the impeller.
6. The blower according to item 5, wherein
   the impeller, the motor, and the flow passage are disposed in that order in a direction along a rotation axis of the impeller.
7. The blower according to item 5 or 6, wherein
   the flow passage is disposed so as to orbit around the motor.
8. The blower according to any one of items 1 to 7, comprising a rectifying member that is provided at a center of the intake port so as to be protruded outwardly from the intake port.
9. The blower according to item 8, wherein
   the rectifying member supports one end of the rotation axis of the impeller.
10. The blower according to any one of items 1 to 9, wherein
   the impeller includes a plurality of blades disposed around the rotation axis and a cover member for covering the plurality of blades toward the partitioning member, with the plurality of blades being open toward the intake port.
11. The blower according to item 10, wherein
   the cover member has a cone surface protruded toward the intake port.
12. The blower according to any one of items 1 to 11, wherein
   the blower is used for a respiration assistance device.
13. A blower comprising:
   a housing having an intake port and a flow passage that communicates with a discharge port;
   an impeller disposed in the housing so as to have a front surface facing the intake port; and
   a partitioning member that is disposed toward a rear surface of the impeller to partition an inside of the housing into a space in which the impeller is disposed and the flow passage, wherein
   the partitioning member forms a slit, which extends along an inner peripheral surface of the housing, between the partitioning member and the inner peripheral surface of the housing, and
   the partitioning member is provided with an air passage connecting between the space and the flow passage.
14. The blower according to item 13, wherein
   the flow passage extends along the slit.
15. The blower according to item 13 or 14, wherein
   the air passage is formed so as to face a rear surface of the impeller.
16. The blower according to any one of items 13 to 15, wherein the slit has a width of 1.0 mm or less.

## Claims

1. A blower (10) comprising:
a housing (11) having an intake port (16) and a flow passage (22) that communicates with a discharge port (17);
an impeller (13) disposed in the housing (11) so as to have a front surface facing the intake port (16); and
a partitioning member (15) that is disposed toward a rear surface of the impeller (13) to partition an inside of the housing (11) into a space (21) in which the impeller (13) is disposed and the flow passage (22), wherein
the partitioning member (15) forms a slit (d), which extends along an inner peripheral surface of the housing (11), between the partitioning member (15) and the inner peripheral surface of the housing (11),
**characterized in that**
the slit (d) has a width (w) of 0.6 mm or less.

2. A blower (10) according to claim 1, furthermore comprising
a rectifying member (12) that is provided at a center of the intake port (16) so as to be protruded outwardly from the intake port (16).

3. A blower (10) according to claim 1, wherein
the flow passage (22) has a sectional shape which is a perfect circle or a shape which is relatively more elongated in a radial direction of the impeller (13).

4. The blower (10) according to any one of claims 1 to 3, wherein
the flow passage (22) extends along the slit (d).

5. The blower (10) according to any one of claims 1 to 4, wherein
the slit (d) has a width (w) of 1.5% or less of an inner peripheral diameter (D) of the housing (11).

6. The blower (10) according to any one of claims 1 to 5, comprising a motor (14) for rotating the impeller (13).

7. The blower (10) according to claim 6, wherein
the impeller (13), the motor (14), and the flow passage (22) are disposed in that order in a direction along a rotation axis (18) of the impeller (13).

8. The blower (10) according to claim 6 or 7, wherein
the flow passage (22) is disposed so as to orbit around the motor (14).

9. The blower (10) according to any one of claims 1 to 8, wherein
the rectifying member (12) supports one end of the rotation axis (18) of the impeller (13).

10. The blower (10) according to any one of claims 1 to 9, wherein the impeller (13) includes a plurality of blades (19) disposed around the rotation axis (18) and a cover member (20) for covering the plurality of blades (19) toward the partitioning member (15), with the plurality of blades (19) being open toward the intake port (16).

11. The blower (10) according to claim 10, wherein
the cover member (20) has a cone surface protruded toward the intake port (16).

12. The blower (10) according to any one of claims 1 to 11, wherein
the blower (10) is used for a respiration assistance device.
